# EUROPEAN PATENT SPECIFICATION

(11) **EP 4 056 136 B1**
(45) Date of publication and mention of the grant of the patent: **08.11.2023**
(21) Application number: 22157097.1
(22) Date of filing: 16.02.2022
(51) Int. Cl.: A61B 18/18

(54) **APPLICATOR NEEDLE FOR MICROWAVE THERMOABLATOR**
APPLIKATORNADEL FÜR MIKROWELLENTHERMOABLATOR
AIGUILLE APPLICATRICE POUR THERMOABLATEUR À MICRO-ONDES

(30) Priority: 11.03.2021 IT 202100005798
(43) Date of publication of application: 14.09.2022
(73) Proprietor: Biomedical - S.r.l., 50144 Firenze (FI) (IT)
(72) Inventor: Castellacci, Pietro, 50022 Greve in Chianti (FI) (IT)
(74) Representative: Fanfani, Stefano

(56) References cited:
- WO-A1-2019/152856
- AU-A1- 2013 249 645
- AU-B2- 2015 264 779
- US-A1- 2020 205 894
- US-A1- 2021 052 317

## Description

### Technical field

The present invention belongs to the field of medical devices used for the treatment and reduction of tumor masses using minimally invasive procedures.

More in detail, the present invention belongs to the field of thermoablators, i.e. devices comprising an antenna shaped like a needle which is inserted into the tissues of the patient and which irradiates non-ionizing microwaves in order to locally raise the temperature of neoplasms.

In particular, the present invention is a microwave thermoablator needle as defined in independent claims 1 and 10.

### Present status of the art

Thermal ablation is a minimally invasive procedure that uses localized hyperthermia to destroy a tumor mass. It can be applied to various organs including the liver, kidneys, lungs and bones and is effective on both primary and secondary tumors (metastases).

The procedure requires the physician to place a thin needle through the skin of the patient to the center of the tumor mass where the needle will heat the tumor due to the high water content of biological tissues and raise the temperature to a level that in a few minutes the neoplastic cells will be destroyed irreversibly, with the added advantage that they do not have to be removed because the body is able to reabsorb them.

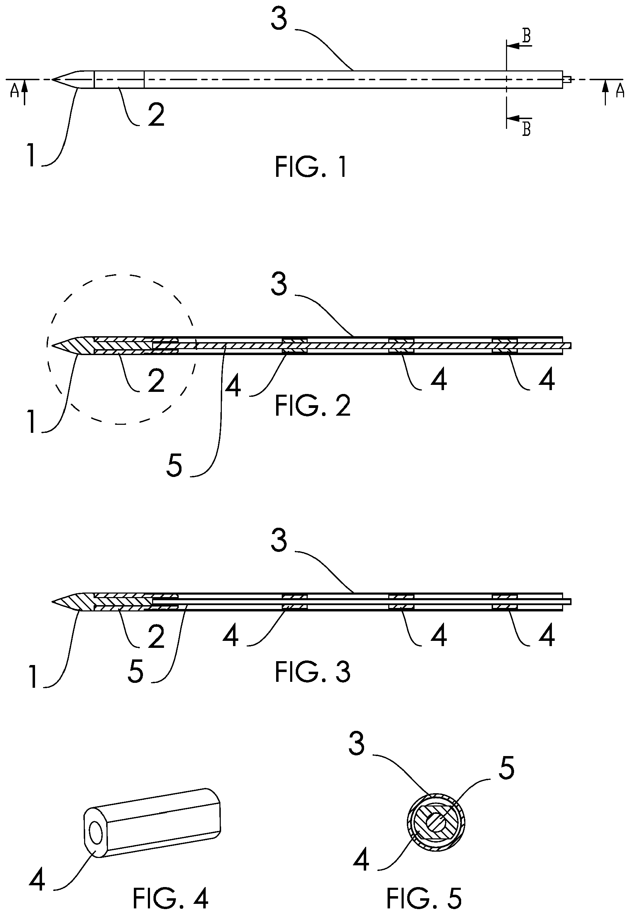

As shown in the sectional views of Fig. 2 and Fig. 3, the applicator needles for thermal ablation comprise an internal conductor and an external conductor, both cylindrical and coaxial to each other. In US7311703, various embodiment solutions are shown.

The internal conductor can be solid or tubular, it is electrically connected with the radiating tip of the needle and must not be in electrical contact with the external tubular conductor, for this reason it is electrically insulated from the radiating tip by means of a sleeve of dielectric material.

The internal conductor and the external conductor must maintain their coaxiality along the entire longitudinal development of the applicator needle.

It should also be emphasized that the electrical insulation between the external and internal tubular conductor significantly affects the electrical operating characteristics of the applicator needle, in particular the characteristic impedance of the device.

The Italian patent IT1420543 teaches to distance the external conductor from the internal conductor by wrapping the latter with a spiral of dielectric material.

The spiral used to ensure the electrical insulation between the internal and external conductor is generally made starting from a cylindrical semi-finished product in PEEK (Polyether-ether-ketone) with a diameter of 0.4 mm. There is, however, the drawback that PEEK has never been characterized for microwave applications. Due to their small size and limited production volumes, these spirals are made by hand-winding the PEEK wire around the internal conductor, which is then inserted into the external conductor, taking great care to ensure that the spiral remains in the intended position.

It is evident that the construction of applicator needles according to the teaching of IT1420543 poses many manufacturing problems, since very often, when the applicator needle has been mounted, the spiral has undergone displacements and its pitch is not constant.

Furthermore, due to the size and limited rigidity of the needle, it is necessary for the coils of the spiral to be fairly close together, resulting in their pitch being too small compared to the wavelength of the electromagnetic wave at 2.45 Ghz (i.e. 12,24 cm), it follows that the dielectric constant of the coaxial transmission varies in an unpredictable way and a power dissipation is also created on the insulating medium.

US 2021/052317 A1 discloses an electrosurgical instrument having a radiating tip with enhanced flexibility.

### Objects and summary of the invention

The problem of providing a microwave thermoablator applicator needle which overcomes the problems of the known art and in particular ensures repetitive and reliable operation, unaffected by assembly, is therefore felt.

A second purpose of the present invention is to provide a microwave thermoablator applicator needle which allows the use of materials characterised for microwaves.

No less important purposes of the present invention are to provide a microwave thermoablator applicator needle whose assembly is simple and repeatable.

These and other purposes which will be clear to the expert in the field are achieved with an applicator needle in which the spacing and centring between the external conductor and the internal conductor are obtained with special spacers of dielectric material.

Preferably, but not exclusively, these spacers are made of PTFE (Polytetrafluoroethylene). PTFE is a well-characterised material for microwave applications and as a dielectric has negligible electromagnetic losses (65 kV/mm dielectric strength and εr=2.1) which remain constant in a very high temperature range.

The PTFE spacers, chosen as an alternative to the spiral, have a minimum axial thickness and, according to a preferred embodiment, are positioned at regular intervals, with a pitch of about 3 cm from each other. In this configuration the transmitted power, very advantageously, is confronted with a dielectric constant comparable to that of vacuum, therefore corresponding to the maximum propagation speed.

A further advantage of PTFE - Polytetrafluoroethylene is its low coefficient of friction, which facilitates sliding when inserted inside the external tubular conductor.

Since the spacers are made from PTFE - Polytetrafluoroethylene based compounds, which is very difficult to glue, forms of connection between the spacers and the conductors are also illustrated in the present patent.

It should be noted that in this patent text with the abbreviation PTFE or with the term Polytetrafluoroethylene we mean both the pure compound and any compound based on Polytetrafluoroethylene and / or any compound based on Polytetrafluoroethylene added in the polymerization phase with modifying monomers; the same terms PTFE or Polytetrafluoroethylene are used to refer to any material which associates a low coefficient of dynamic friction with high dielectric properties, similar to those of PTFE.

### Brief description of the drawings

**Fig. 1** shows a side view of an assembled needle (1) .
**Fig. 2** shows section A-A of the needle shown in Fig. 1 in which the internal conductor (5) is shaped like a solid cylinder, the section is taken according to a longitudinal plane.
**Fig. 3** shows the section A-A of the needle shown in Fig. 1 in which the internal conductor (5) is shaped like a tubular, the section is taken according to a longitudinal plane.
**Fig. 4** shows a perspective view of a spacer (4).
**Fig. 5** shows a section of the needle (1) according to plan B-B.

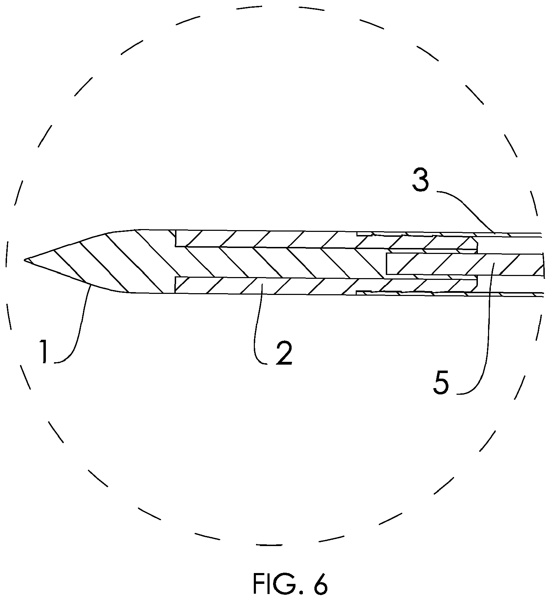

**Fig. 6** shows an enlarged view of the radiating tip of the needle (1).

### Detailed description of preferred embodiments of the invention

The applicator needle for thermoablators comprises a radiating tip (1) of conductive material, axisymmetrical, the rear portion of which has an outer diameter smaller than that of the central portion, and is slidingly engaged in a tubular sleeve (2) of insulating material.

The latter is shaped as a cylinder, the front portion of which has an inner diameter equal to the outer diameter of the rear portion of the radiating tip (1) and an outer diameter equal to the outer diameter of the central portion of the radiating tip (1); the rear portion of the tubular sleeve (2) has an outer diameter equal to the inner diameter of the outer tubular conductor (3) and slides irreversibly into the latter.

In the rear portion of the radiating tip (1), a cylindrical cavity is axially defined, the inner diameter of which is equal to the outer diameter of the internal conductor (5) whose distal end is housed there in electrical continuity.

All components of the applicator needle are coaxial with the longitudinal axis of the needle itself.

The centering and separation between the internal conductor (5) and the external tubular conductor (3) is made using a plurality of cylindrical spacers (4), in each of which is defined a cylindrical cavity whose inner diameter is equal to the outer diameter of the internal conductor (5) and whose outer surface is partly a first cylindrical surface whose diameter is equal to the inner diameter of the external conductor (3) and partly a second surface, closer to the longitudinal axis of said first cylindrical surface, so as to be loose with respect to the inner diameter of the external conductor (3).

To allow the flow of gases inside the needle and to permit easy assembly, it is important that only part of the outer surface of the spacers (4) is in contact with the inner surface of the external tubular conductor (3).

The spacers (4) are mounted on the internal conductor (5) by sliding them onto it; since it is not possible to use gluing to hold them in position, advantageously the assembly takes place with a slight interference, in other words, the inner diameter of their axial cavity is slightly smaller than the outer diameter of the internal conductor (5) so that their insertion and sliding requires a slight forcing which serves to keep them in position when the internal conductor (5), with the spacers (4) already mounted, is slid inside the external tubular conductor (3).

The spacers (4), and preferably also the tubular sleeve (2), are made with compounds based on PTFE - Polytetrafluoroethylene added in the polymerization phase with modifying monomers.

A preferred embodiment of the invention provides that the distal end of the internal conductor (5) is made integral with the interior of the cylindrical cavity axially defined in the rear portion of the radiating tip (1) by means of a laser welding.

In order to facilitate the insertion and extraction of the needle from the patient's tissues, a particularly performing solution provides that, after having fixed the distal end of the internal conductor (5) inside the cylindrical cavity defined in the rear portion of the radiating tip (1), the latter will be coated with a thin layer of PTFE to decrease its friction coefficient; this surface coating, also called tefloning, continues only for a few centimetres on the outer surface of the internal conductor (5). It is also advantageous to coat the outer surface of the external conductor (3) with a thin layer of PTFE, even if only partially.

In order to improve its electrical characteristics, the portion of the outer surface of the internal conductor (5) which is not coated with PTFE may be coated with a thin layer of silver.

As mentioned above, the PTFE layer that covers the radiating tip (1) also continues for a short distance on the outer surface of the internal conductor (5) and, thanks to the non-stick properties of PTFE, the silver does not adhere to the latter, thus avoiding the risk that, in proximity of the radiating tip (1), the silver coating could put the internal conductor (5) and the external conductor (3) in electrical continuity.

It should be noted that in the present patent text, the term "silver" means any material, even non-metallic, having high electrical conductivity characteristics.

In the case of very thin applicator needles the fabrication and correct positioning of the spacers (4) on the internal conductor (5) becomes particularly difficult.

The external conductor of the 18-gauge needles has an outer diameter of 1,27 mm and an inner diameter of 1,05 mm, while the internal conductor has an outer diameter of only 0,40 mm and an inner diameter of 0,20 mm; thus the thickness of the cylindrical gap defined between the two conductors is only 0,325 mm.

In these cases, a different embodiment of the invention is advantageously used, which instead of spacers uses a heat-shrinkable tube of PTFE - Polytetrafluoroethylene which is slipped over said internal conductor (5), to be then heated until it adheres to the outer surface of said internal conductor before being inserted inside the external conductor (3).

## Claims

1. Applicator needle for thermoablators comprising:
- a radiant tip (1) of conductive material, axially symmetrical, with a central portion and a rear portion in which a cylindrical cavity is axially defined;
- an internal conductor (5), cylindrical externally, the front end of which fits slidingly into said cylindrical cavity of said radiant tip (1) with which it is in electrical contact;
- a tubular sleeve (2) of dielectric material, which houses inside the rear portion of said radiant tip (1) and whose inner diameter is equal to the outer diameter of the latter, the inner diameter of the front portion of said tubular sleeve (2) is equal to the outer diameter of the central portion of said radiant tip (1) and is greater than the outer diameter of the rear portion of said tubular sleeve (2);
- an external tubular conductor (3) whose outer diameter is equal to the outer diameter of the central portion of said radiant tip (1) and to the outer diameter of said front portion of said tubular sleeve (2); the front end of said external tubular conductor (3) has an inner diameter equal to the outer diameter of said rear portion of said tubular sleeve (2) on which it is slidingly engaged in an irreversible manner;
**characterized in that** it comprises a plurality of spacers (4) of dielectric material, in each of which a cylindrical axial cavity is defined whose inner diameter is equal to the outer diameter of said internal conductor (5) which is slidably received therein and to which they are integrally connected, and whose outer surface is in part a first cylindrical surface whose diameter is equal to the inner diameter of the external conductor (3) and in part is a second surface, closer to the longitudinal axis of said first cylindrical surface, so as to be only partially in contact with the inner surface of said external conductor (3).

2. Applicator needle according to preceding claim 1 **characterized in that** said spacers (4) are equidistant from each other.

3. Applicator needle according to preceding claim 2 **characterized in that** the distance between said spacers (4) is comprised between 2 and 4 centimetres.

4. Applicator needle according to one of the preceding claims **characterized in that** said spacers (4) and / or said tubular sleeve (2) are made with PTFE - Polytetrafluoroethylene based compounds.

5. Applicator needle according to one of the preceding claims **characterized in that** said rear portion of said tubular sleeve (2) and said inner surface of the front end of said external tubular conductor (3) comprise anti-loosening means.

6. Applicator needle according to preceding claim 5 **characterized in that** said anti-loosening means comprise a plurality of circumferential reliefs which protrude from the outer surface of said rear portion of said tubular sleeve (2).

7. Applicator needle according to one of the preceding claims **characterized in that** the assembly of said spacers (4) on said internal conductor (5) takes place with slight interference, wherein the inner diameter of the cylindrical axial cavity of each spacer is slightly smaller than the outer diameter of the inner conductor (5) such that the said spacers (4) are kept in position.

8. Applicator needle according to one of the preceding claims **characterized in that** the outer surface of said radiant tip (1) and of said external tubular conductor (3) are coated with a thin layer of PTFE - Polytetrafluoroethylene.

9. Applicator needle according to one of the preceding claims **characterized in that** the outer surface of said internal conductor (5) is at least partially coated with a thin layer of silver.

10. Applicator needle for thermoablators comprising:
- a radiant tip (1) of conductive material, axially symmetrical, with a central portion and a rear portion in which a cylindrical cavity is axially defined;
- an internal conductor (5), cylindrical externally, the front end of which fits slidingly into said cylindrical cavity of said radiant tip (1) with which it is in electrical contact;
- a tubular sleeve (2) of dielectric material, which houses inside the rear portion of said radiant tip (1) and whose inner diameter is equal to the outer diameter of the latter, the outer diameter of the front portion of said tubular sleeve (2) is equal to the outer diameter of the central portion of said radiant tip (1) and is greater than the outer diameter of the rear portion of said tubular sleeve (2);
- an external tubular conductor (3) whose outer diameter is equal to the outer diameter of the central portion of said radiant tip (1) and to the outer diameter of said front portion of said tubular sleeve (2); the front end of said external tubular conductor (3) has an inner diameter equal to the outer diameter of said rear portion of said tubular sleeve (2) on which it is slidingly engaged in an irreversible manner;
**characterized in that** said internal conductor (5) is slidably inserted inside a heat-shrinkable PTFE tube made adherent to the outer surface of said internal conductor (5) before inserting it inside said external conductor (3).

## Patentansprüche

1. Applikatornadel für Thermoablatoren, bestehend aus:
- einer strahlenden Spitze (1) aus leitfähigem Material, axialsymmetrisch, mit einem mittleren Teil und einem hinteren Teil, in dem ein zylindrischer Hohlraum axial definiert ist;
- einem inneren, außen zylindrischen Leiter (5), dessen vorderes Ende gleitend in den zylindrischen Hohlraum der strahlenden Spitze (1) eingreift, mit der er in elektrischem Kontakt steht;
- einer röhrenförmigen Hülse (2) aus dielektrischem Material, die in ihrem Inneren den hinteren Teil der strahlenden Spitze (1) unterbringt und deren Innendurchmesser gleich dem Außendurchmesser der letzteren ist, wobei der Innendurchmesser des vorderen Teils der röhrenförmigen Hülse (2) gleich dem Außendurchmesser des mittleren Teils der strahlenden Spitze (1) ist und größer als der Außendurchmesser des hinteren Teils der röhrenförmigen Hülse (2) ist;
- einem äußeren rohrförmigen Leiter (3), dessen Außendurchmesser gleich dem Außendurchmesser des mittleren Teils der strahlenden Spitze (1) und dem Außendurchmesser des vorderen Teils der rohrförmigen Hülse (2) ist; das vordere Ende des äußeren rohrförmigen Leiters (3) einen Innendurchmesser aufweist, der gleich dem Außendurchmesser des hinteren Teils der rohrförmigen Hülse (2) ist, auf der er irreversibel gleitend aufliegt;
**gekennzeichnet dadurch, dass** sie eine Vielzahl von Abstandshaltern (4) aus dielektrischem Material umfasst, in denen jeweils ein zylindrischer axialer Hohlraum definiert ist, dessen Innendurchmesser gleich dem Außendurchmesser des Innenleiters (5) ist, der darin gleitend aufgenommen ist und mit dem sie fest verbunden sind, und dessen Außenfläche teilweise eine erste zylindrische Fläche ist, deren Durchmesser gleich dem Innendurchmesser des Außenleiters (3) ist, und teilweise eine zweite Fläche ist, die näher an der Längsachse der ersten zylindrischen Fläche liegt, so dass sie nur teilweise in Kontakt mit der Innenfläche des Außenleiters (3) ist.

2. Applikatornadel nach dem vorhergehenden Anspruch 1, **dadurch gekennzeichnet, dass** die Abstandshalter (4) gleich weit voneinander entfernt sind.

3. Applikatornadel nach dem vorhergehenden Anspruch 2, **dadurch gekennzeichnet, dass** der Abstand zwischen den Abstandshaltern (4) zwischen 2 und 4 Zentimeter beträgt.

4. Applikatornadel nach einem der vorhergehenden Ansprüche, **dadurch gekennzeichnet, dass** die Abstandshalter (4) und / oder die röhrenförmige Hülse (2) aus Verbindungen auf Basis von PTFE (Polytetrafluorethylen) hergestellt sind.

5. Applikatornadel nach einem der vorhergehenden Ansprüche, **dadurch gekennzeichnet, dass** der hintere Teil der rohrförmigen Hülse (2) und die Innenfläche des vorderen Endes des äußeren rohrförmigen Leiters (3) Mittel zur Verhinderung von Lockerungen aufweisen.

6. Applikatornadel nach dem vorhergehenden Anspruch 5, **dadurch gekennzeichnet, dass** die Anti-Locker-Mittel eine Vielzahl von Umfangsreliefs umfassen, die von der Außenfläche des hinteren Teils der rohrförmigen Hülse (2) vorstehen.

7. Applikatornadel nach einem der vorhergehenden Ansprüche, **dadurch gekennzeichnet, dass** die Montage der Abstandshalter (4) auf dem Innenleiter (5) mit leichtem Übermaß erfolgt, wobei der Innendurchmesser des zylindrischen axialen Hohlraums jedes Abstandshalters etwas kleiner ist als der Außendurchmesser des Innenleiters (5), so dass die Abstandshalter (4) in Position gehalten werden.

8. Applikatornadel nach einem der vorhergehenden Ansprüche, **dadurch gekennzeichnet, dass** die äußere Oberfläche der strahlenden Spitze (1) und des äußeren rohrförmigen Leiters (3) mit einer dünnen Schicht aus PTFE - Polytetrafluorethylen - überzogen ist.

9. Applikatornadel nach einem der vorhergehenden Ansprüche, **dadurch gekennzeichnet, dass** die Außenfläche des Innenleiters (5) zumindest teilweise mit einer dünnen Silberschicht überzogen ist.

10. Applikatornadel für Thermoablatoren, bestehend aus:
- einer strahlenden Spitze (1) aus leitfähigem Material, axialsymmetrisch, mit einem mittleren Teil und einem hinteren Teil, in dem ein zylindrischer Hohlraum axial definiert ist;
- einem inneren, außen zylindrischen Leiter (5), dessen vorderes Ende gleitend in den zylindrischen Hohlraum der strahlenden Spitze (1) eingreift, mit der er in elektrischem Kontakt steht;
- einer röhrenförmigen Hülse (2) aus dielektrischem Material, die in ihrem Inneren den hinteren Teil der strahlenden Spitze (1) unterbringt und deren Innendurchmesser gleich dem Außendurchmesser der letzteren ist, wobei der Außendurchmesser des vorderen Teils der röhrenförmigen Hülse (2) gleich dem Außendurchmesser des mittleren Teils der strahlenden Spitze (1) ist und größer als der Außendurchmesser des hinteren Teils der röhrenförmigen Hülse (2) ist;
- einem äußeren rohrförmigen Leiter (3), dessen Außendurchmesser gleich dem Außendurchmesser des mittleren Teils der strahlenden Spitze (1) und dem Außendurchmesser des vorderen Teils der rohrförmigen Hülse (2) ist; das vordere Ende des äußeren rohrförmigen Leiters (3) einen Innendurchmesser aufweist, der gleich dem Außendurchmesser des hinteren Teils der rohrförmigen Hülse (2) ist, auf der er irreversibel gleitend aufliegt;
**dadurch gekennzeichnet, dass** der Innenleiter (5) gleitend in einen wärmeschrumpfenden PTFE-Schlauch eingeführt ist, der an der Außenfläche des Innenleiters (5) haftet, bevor er in den Außenleiter (3) eingeführt wird.

## Revendications

1. Aiguille applicatrice pour thermoablateurs comprenant:
- une pointe rayonnante (1) en matériau conducteur, axialement symétrique, avec une partie centrale et une partie arrière dans laquelle une cavité cylindrique est axialement définie ;
- un conducteur interne (5), cylindrique extérieurement, dont l'extrémité avant s'ajuste de manière coulissante dans ladite cavité cylindrique de ladite pointe rayonnante (1) avec laquelle il est en contact électrique ;
- un manchon tubulaire (2) en matériau diélectrique, qui loge à l'intérieur la partie arrière de ladite pointe rayonnante (1) et dont le diamètre interne est égal au diamètre externe de cette dernière, le diamètre interne de la partie avant dudit manchon tubulaire (2) est égal au diamètre externe de la partie centrale de ladite pointe rayonnante (1) et est supérieur au diamètre externe de la partie arrière dudit manchon tubulaire (2) ;
- un conducteur tubulaire externe (3) dont le diamètre externe est égal au diamètre externe de la partie centrale de ladite pointe rayonnante (1) et au diamètre externe de ladite partie avant dudit manchon tubulaire (2) ; l'extrémité avant dudit conducteur tubulaire externe (3) a un diamètre interne égal au diamètre externe de ladite partie arrière dudit manchon tubulaire (2) sur lequel il est engagé de manière coulissante de façon irréversible ;
**caractérisé en ce qu'**il comprend une pluralité d'entretoises (4) en matériau diélectrique, dans chacune desquelles une cavité axiale cylindrique est définie dont le diamètre interne est égal au diamètre externe dudit conducteur interne (5) qui y est reçu de manière coulissante et auxquelles elles sont intégralement reliées, et dont la surface externe est en partie une première surface cylindrique dont le diamètre est égal au diamètre interne du conducteur externe (3) et en partie est une deuxième surface, plus proche de l'axe longitudinal de ladite première surface cylindrique, de manière à être en contact seulement partiellement avec la surface interne dudit conducteur externe (3).

2. Aiguille applicatrice selon la revendication 1 précédente, **caractérisée en ce que** lesdites entretoises (4) sont équidistantes les unes des autres.

3. Aiguille applicatrice selon la revendication 2 précédente, **caractérisée en ce que** la distance entre lesdites entretoises (4) est comprise entre 2 et 4 centimètres.

4. Aiguille applicatrice selon l'une des revendications précédentes, **caractérisée en ce que** lesdites entretoises (4) et/ou ledit manchon tubulaire (2) sont fabriqués avec des composés à base de PTFE - Polytétrafluoroéthylène.

5. Aiguille applicatrice selon l'une des revendications précédentes, **caractérisée en ce que** ladite partie arrière dudit manchon tubulaire (2) et ladite surface interne de l'extrémité avant dudit conducteur tubulaire externe (3) comprennent des moyens anti-desserrage.

6. Aiguille applicatrice selon la revendication 5 précédente, **caractérisée en ce que** lesdits moyens anti-desserrage comprennent une pluralité de reliefs circonférentiels qui font saillie à partir de la surface externe de ladite partie arrière dudit manchon tubulaire (2).

7. Aiguille applicatrice selon l'une des revendications précédentes, **caractérisée en ce que** l'assemblage desdites entretoises (4) sur ledit conducteur interne (5) se fait avec une légère interférence, où le diamètre interne de la cavité axiale cylindrique de chaque entretoise est légèrement plus petit que le diamètre externe du conducteur interne (5) de sorte que lesdites entretoises (4) sont maintenues en position.

8. Aiguille applicatrice selon l'une des revendications précédentes, **caractérisée en ce que** la surface externe de ladite pointe rayonnante (1) et dudit conducteur tubulaire externe (3) sont recouvertes d'une fine couche de PTFE - Polytétrafluoroéthylène.

9. Aiguille applicatrice selon l'une des revendications précédentes, **caractérisée en ce que** la surface externe dudit conducteur interne (5) est au moins partiellement recouverte d'une fine couche d'argent.

10. Aiguille applicatrice pour thermoablateurs comprenant:
- une pointe rayonnante (1) en matériau conducteur, axialement symétrique, avec une partie centrale et une partie arrière dans laquelle une cavité cylindrique est axialement définie ;
- un conducteur interne (5), cylindrique extérieurement, dont l'extrémité avant s'ajuste de manière coulissante dans ladite cavité cylindrique de ladite pointe rayonnante (1) avec laquelle il est en contact électrique ;
- un manchon tubulaire (2) en matériau diélectrique, qui loge à l'intérieur la partie arrière de ladite pointe rayonnante (1) et dont le diamètre interne est égal au diamètre externe de cette dernière, le diamètre externe de la partie avant dudit manchon tubulaire (2) est égal au diamètre externe de la partie centrale de ladite pointe rayonnante (1) et est supérieur au diamètre externe de la partie arrière dudit manchon tubulaire (2) ;
- un conducteur tubulaire externe (3) dont le diamètre externe est égal au diamètre externe de la partie centrale de ladite pointe rayonnante (1) et au diamètre externe de ladite partie avant dudit manchon tubulaire (2) ; l'extrémité avant dudit conducteur tubulaire externe (3) a un diamètre interne égal au diamètre externe de ladite partie arrière dudit manchon tubulaire (2) sur lequel il est engagé de manière coulissante de façon irréversible ;
**caractérisé en ce que** ledit conducteur interne (5) est inséré de manière coulissante à l'intérieur d'un tube en PTFE thermorétractable rendu adhérent à la surface externe dudit conducteur interne (5) avant son insertion à l'intérieur dudit conducteur tubulaire externe (3).
